# EUROPEAN PATENT APPLICATION

(11) **EP 4 160 277 A1**
(43) Date of publication of application: **05.04.2023**
(21) Application number: 21814516.7
(22) Date of filing: 24.05.2021
(51) Int. Cl.: G01V 5/00, G01N 23/046

(54) **CT SCANNING DEVICE**

(30) Priority: 29.05.2020 CN 202010482062
(71) Applicant: Tsinghua University, Beijing 100084 (CN); Nuctech Company Limited, TongFang Building Shuangqinglu Haidian District Beijing 100084 (CN)
(72) Inventor: CHEN, Zhiqiang, Beijing 100084 (CN); ZHANG, Li, Beijing 100084 (CN); HUANG, Qingping, Beijing 100084 (CN); HONG, Mingzhi, Beijing 100084 (CN); ZHANG, Liguo, Beijing 100084 (CN); LI, Guipei, Beijing 100084 (CN)
(74) Representative: Gunzelmann, Rainer
(86) International application number: PCT/CN2021/095517
(87) International publication number: WO 2021/238862

(57) **Abstract**

There is provided a CT scanning apparatus (100), including: a support frame (1); a slip ring (2); a driving mechanism (3) configured to drive the slip ring (2) to rotate with respect to the support frame (1); at least two annular rails (4) mounted on an outer periphery of the slip ring; and at least two groups of support devices (5), each group of support devices (5) includes at least two roller assemblies (51) surrounding the annular rail (4) and configured to support the slip ring (2). Each roller assembly (51) includes a roller (511), the roller (511) is mounted on the support frame (1) and engaged with the annular rail to support the slip ring (2). A roller (511) of at least one roller assembly (51) of the at least two roller assemblies (51) is engaged with the annular rail (4) to prevent a movement of the slip ring (2) in an axial direction. A cantilever structure used to support the slip ring may be omitted by using a plurality of rollers (511) having a small external diameter to support the slip ring (2), and the rollers (511) may be easily replaced when being damaged.

## Description

### CROSS REFERENCE TO RELATED APPLICATION(S)

This application claims priority to Chinese Patent Application No. 202010482062.6, filed on May 29, 2020, the content of which is incorporated herein by reference in its entirety.

### TECHNICAL FIELD

Embodiments of the present disclosure relate to a CT scanning apparatus, and in particular to a CT scanning apparatus having a slip ring.

### BACKGROUND

A traditional CT scanning apparatus mainly includes: a scanning device, a computer system, a power supply and accessory apparatuses. The scanning device includes a slip ring (rotating gantry), an X-ray tube and a detector which are mounted on the slip ring. An X-ray beam generated by the X-ray tube performs a transverse scanning on an inspected object such as a human body or a cargo through a collimator. Each detector receives an attenuation signal from an X-ray penetrating a human tissue or a cargo, converts the attenuation signal to an electrical signal, and then the electrical signal is converted into a digital signal (i.e., an original data) by an analog-to-digital conversion, and the digital signal is then stored in a memory. A magnitude of attenuation of the X-ray penetrating the inspected object is a function of a density of a substance through which a radiation beam passes. An attenuated X-ray is detected, and an X-ray photo image of the inspected object is generated to illustrate a result of the inspection.

In an existing CT scanning apparatus, a single large bearing is used as a rotating support for a slip ring. The slip ring, the X-ray tube and the detector are rotatably mounted on the large bearing, and a driving mechanism drives the slip ring to rotate through a multi-ribbed belt. A transmission channel for transmitting an inspected object passes through the large bearing and the slip ring, and the inspected object is detected while moving in the transmission channel.

In another existing CT scanning apparatus, the slip ring is rotatably supported by several small bearing wheels, a motor is used to directly drive one of the small bearing wheels, and a rotary motion of the slip ring is driven by the friction wheel drive principle. Small bearing dampers are provided on two end surfaces of the slip ring to limit an axial displacement of the slip ring. A transmission channel for transmitting an inspected object passes through a large bearing and the slip ring, and the inspected object is detected while moving in the transmission channel.

### SUMMARY

The objective of the present disclosure is to solve at least one aspect of the above problems and defects in the related art.

According to an embodiment in an aspect of the present disclosure, a CT scanning apparatus is provided, including: a support frame; a slip ring; at least two annular rails mounted on an outer periphery of the slip ring; a driving mechanism configured to drive the slip ring to rotate with respect to the support frame; and at least two groups of support devices, wherein each group of support devices comprises at least two roller assemblies surrounding the annular rail and configured to support the slip ring, wherein each roller assembly comprises at least one roller, and the at least one roller is mounted on the support frame and engaged with the annular rail so as to support the slip ring, and wherein a roller of at least one roller assembly of the at least two roller assemblies is engaged with the annular rail to prevent a movement of the slip ring in an axial direction.

According to an embodiment of the present disclosure, the roller of the at least one roller assembly has an annular recess surrounding an outer periphery of the roller, and the annular rail is at least partially engaged into the recess.

According to an embodiment of the present disclosure, the annular rail has a rail recess arranged in a circumferential direction, and the roller of the at least one roller assembly is at least partially engaged into the rail recess.

According to an embodiment of the present disclosure, the recess of each roller has a substantially V-shaped cross section, and a cross section of the annular rail has a shape cooperating with the recess.

According to an embodiment of the present disclosure, an included angle between two sides of the concave having the substantially V-shape is 85 to 95 degrees, preferably 90 degrees.

According to an embodiment of the present disclosure, each roller assembly further comprises: a base mounted on the support frame; and a support shaft fixedly mounted on the base, wherein the roller is rotatably mounted on the support shaft.

According to an embodiment of the present disclosure, each group of support devices comprises four roller assemblies arranged at equal intervals on the outer periphery of the slip ring.

According to an embodiment of the present disclosure, the base of each roller assembly is mounted with a roller.

According to an embodiment of the present disclosure, in the four bases of the four roller assemblies, two bases located on an upper portion of an axis of the slip ring are respectively mounted with one roller, and two bases located on a lower portion of the axis of the slip ring are respectively mounted with two rollers.

According to an embodiment of the present disclosure, in the four bases of the four roller assemblies, two bases located on an upper portion of an axis of the slip ring are respectively mounted with an elastic mechanism, and the elastic mechanisms are configured so that the rollers mounted on the two bases elastically abut on the annular rail.

According to an embodiment of the present disclosure, an annular mounting seat is provided at one end of the slip ring, and the driving mechanism is configured to drive the mounting seat to rotate.

According to an embodiment of the present disclosure, the driving mechanism comprises: a stator assembly mounted on the support frame; and a rotor assembly mounted on the mounting seat, wherein the stator assembly is electromagnetically coupled with the rotor assembly so as to drive the rotor assembly to rotate with respect to the stator assembly.

According to an embodiment of the present disclosure, the stator assembly comprises: a stator mounted on the support frame, wherein the stator has a cylinder shape; and an induction coil wound on the stator, and wherein the rotor assembly comprises: a rotor having a cylinder shape, wherein one end of the rotor is mounted on the mounting seat and located on an inner side of the stator; and a permanent magnet mounted on an outer side of the rotor and electromagnetically coupled with the induction coil.

According to an embodiment of the present disclosure, one of the two annular rails is mounted on the mounting seat.

According to an embodiment of the present disclosure, at least two lubricating mechanisms are provided on the support frame, and the lubricating mechanism is configured to apply a lubricant to the annular rail.

According to an embodiment of the present disclosure, each lubricating mechanism comprises: a container mounted on the support frame and configured to contain the lubricant; an applicator configured to apply the lubricant to the annular rail; and a transmission pipe connected between the container and the applicator so as to transmit the lubricant in the container to the applicator.

According to an embodiment of the present disclosure, the lubricating mechanism further comprises a regulator configured to regulate a flow of the lubricant.

According to an embodiment of the present disclosure, the lubricating mechanism further comprises a support stand, the support stand is mounted on the support frame, and the applicator is mounted on the support stand.

According to an embodiment of the present disclosure, a buffer layer is provided on a surface of at least one of the annular rail and the roller.

### BRIEF DESCRIPTION OF THE DRAWINGS

FIG. 1 shows a simplified schematic diagram of a CT scanning apparatus according to an exemplary embodiment of the present disclosure;
FIG. 2 shows a principle schematic diagram of a CT scanning apparatus according to an exemplary embodiment of the present disclosure;
FIG. 3 shows a side view of a partial section of a CT scanning apparatus of an exemplary embodiment of the present disclosure;
FIG. 4 shows an enlarged schematic diagram of part A shown in FIG. 3;
FIG. 5 shows an enlarged schematic diagram of part B shown in FIG. 3;
FIG. 6 shows a simplified schematic diagram of a cooperation of a roller with an annular rail according to an exemplary embodiment of the present disclosure;
FIG. 7 shows a front view of the CT scanning apparatus shown in FIG. 3; and
FIG. 8 shows an enlarged schematic view of part C shown in FIG. 7.

### DETAILED DESCRIPTION OF EMBODIMENTS

Technical solutions in the embodiments of the present disclosure will be clearly and completely described below with reference to the accompanying drawings in the embodiments of the present disclosure. Obviously, the described embodiments are only a part, not all of the embodiments of the present disclosure. The following description of at least one exemplary embodiment is actually illustrative only and is in no way intended to limit the present disclosure and an application or use of the present disclosure. Based on the embodiments of the present disclosure, all other embodiments obtained by those of ordinary skill in the art without creative work fall within the protection scope of the present disclosure.

In the following detailed descriptions, for ease of explanation, various specific details are set forth in order to provide a comprehensive understanding of the embodiments of the present disclosure. Obviously, however, one or more embodiments may be implemented without these specific details. In other circumstances, well-known structures and devices are shown in a diagram form to simplify the drawings. Technologies, methods, and apparatuses known to those of ordinary skill in the related art may not be discussed in detail, but where appropriate, such technologies, methods, and apparatuses should be considered as a part of the authorized specification.

In the descriptions of the present disclosure, it should be understood that orientations or positional relationships indicated by orientation words such as "front, rear, upper, lower, left, right", "transverse, vertical, perpendicular, horizontal" and "top, bottom" are usually orientations or positional relationships based on the drawings, and are based on a travel direction of a vehicle. They are only used for ease of describing the present disclosure and simplifying the description. Unless stated in contrary, these orientation words do not indicate and imply that a device or an element referred to necessarily has a specific orientation or is configured and operated in a specific orientation. Therefore, they should not be construed as a limitation on the protection scope of the present disclosure. The orientation words "inner" and "outer" refer to an inside and an outside relative to a profile of each component itself.

In the descriptions of the present disclosure, it should be understood that words such as "first" and "second" used to define components are only for ease of distinguishing the corresponding components. Unless otherwise stated, the above-mentioned words have no special meaning, and therefore, they may not be construed as a limitation on the protection scope of the present disclosure.

According to a general inventive concept of the present disclosure, there is provided a CT scanning apparatus, including: a support frame; a slip ring; a driving mechanism configured to drive the slip ring to rotate with respect to the support frame; at least two annular rails mounted on an outer peripheral side of the slip ring; and at least two groups of support devices, each group includes at least two roller assemblies surrounding the annular rail and configured to support the slip ring. Each roller assembly includes a roller, the roller is mounted on the support frame and engaged with the annular rail to support the slip ring. A roller of at least one roller assembly of the at least two roller assemblies is engaged with the annular rail so as to prevent a movement of the slip ring in an axial direction.

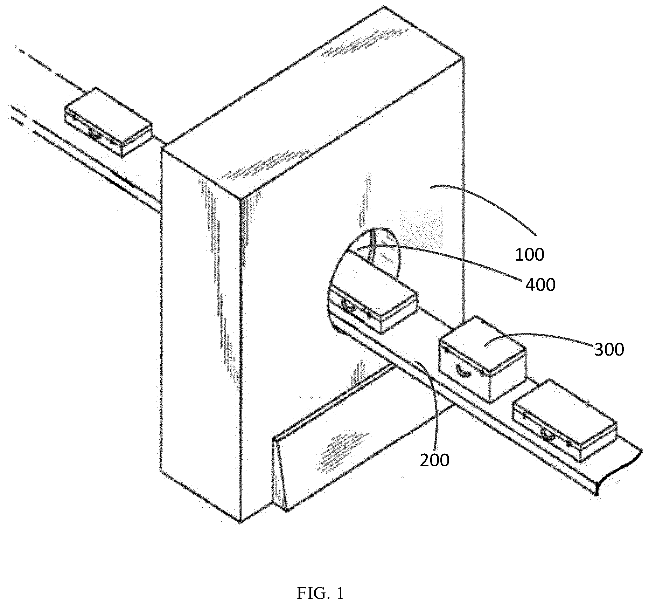

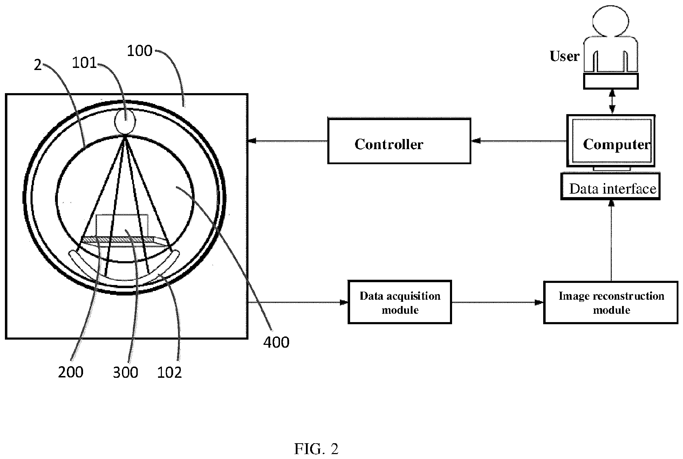

FIG. 1 shows a simplified schematic diagram of a CT scanning apparatus according to an exemplary embodiment of the present disclosure, and FIG. 2 shows a principle schematic diagram of a CT (computed tomography) scanning apparatus according to an exemplary embodiment of the present disclosure.

In an exemplary embodiment, referring to FIG. 1 and FIG. 2, a CT scanning apparatus 100 is suitable for inspecting a presence of prohibited items such as drugs, explosives, and flammables in an object 300 such as a package, a suitcase and a handbag at a place such as a station, an airport a dock and the like. The CT scanning apparatus 100 includes: an inspection channel 400; a transmission device 200 transmitting the inspected object 300 in the inspection channel 400; and a scanning device configured to inspect the object 200 transmitted by the conveying device 200. The transmission device 200 includes a transmission belt 201 for carrying the inspected object and a driving roller 202 for driving the transmission belt to move.

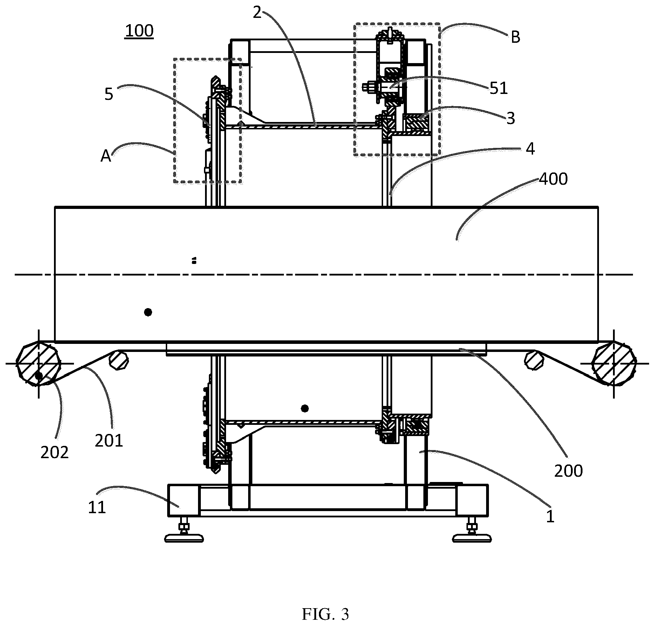

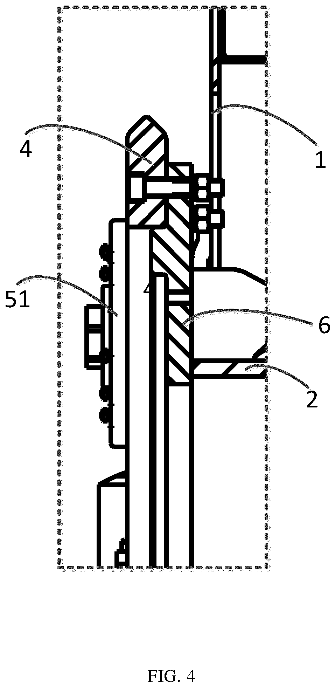

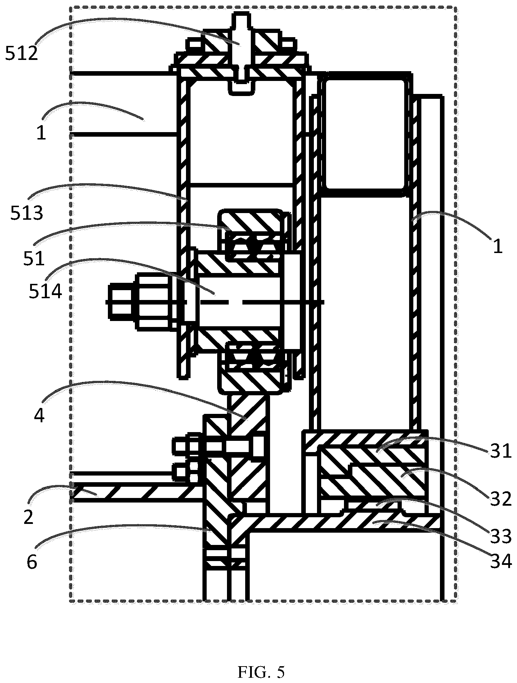

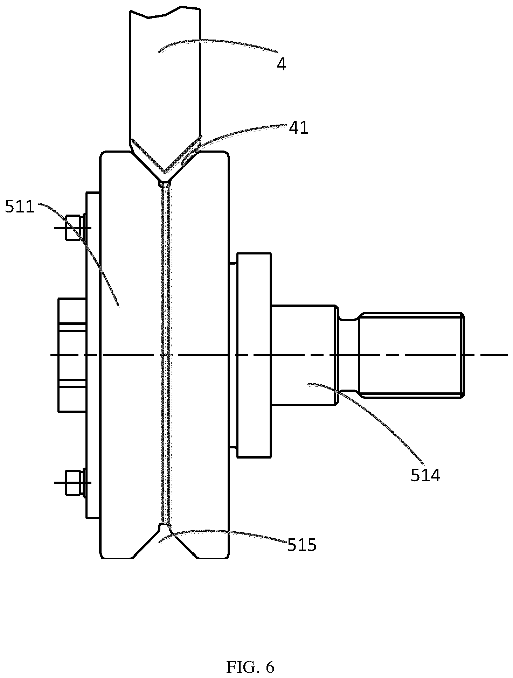

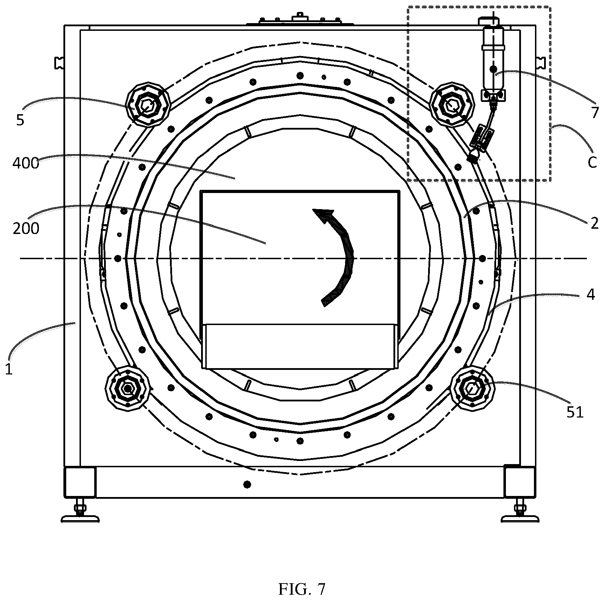

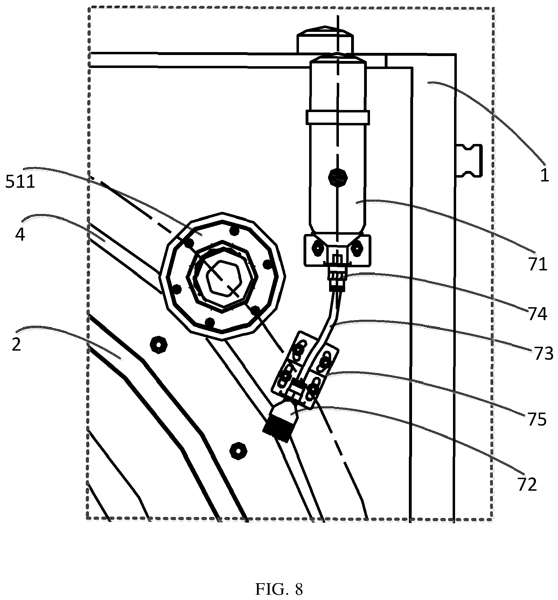

FIG. 3 shows a side view of a partial section of a CT scanning apparatus of an exemplary embodiment of the present disclosure; FIG. 4 shows an enlarged schematic diagram of part A shown in FIG. 3; FIG. 5 shows an enlarged schematic diagram of part B shown in FIG. 3; FIG. 6 shows a simplified schematic diagram of a cooperation of a roller with an annular rail according to an exemplary embodiment of the present disclosure; FIG. 7 shows a front view of the CT scanning apparatus shown in FIG. 3; and FIG. 8 shows an enlarged schematic view of part C shown in FIG. 7.

In an exemplary embodiment, referring to FIG. 1 to FIG. 8, the CT scanning apparatus 100 further includes: a support frame 1 having an outer profile of a substantially cubic shape, and the support frame 1 is supported on a pedestal 11; a slip ring 2 rotatably supported in the support frame 1, and the inspection passage 400 penetrates the slip ring 2; a driving mechanism 3 configured to drive the slip ring 2 to rotate with respect to the support frame 2; at least two annular rails 4 mounted on an outer peripheral side of the slip ring 2; and at least two groups of support devices 5, each group of the support devices 5 includes at least two roller assemblies 51 surrounding the annular rail 4 and configured to support the slip ring 2. Each roller assembly 51 includes at least one roller 511, the roller 511 is mounted on the support frame 1 and engaged with the annular rail 4 so as to support the slip ring 2. A roller 511 of at least one roller assembly of the at least two roller assemblies 51 is engaged with the annular rail 4 to prevent a movement of the slip ring 2 in an axial direction. The scanning device includes the slip ring (rotating gantry) 2 and an X-ray tube 101 and a detector array 102 which are mounted on the slip ring 2. In this way, the support device 5 is suitable for supporting the slip ring 2, and at the same time, the at least one roller assembly in the support device 5 also has a function of preventing the movement of the slip ring 2 in the axial direction.

During an inspection of the object 300, a controller receives an operation instruction input by a user through a computer at a workstation, and controls an action of the driving mechanism 3 according to the operation instruction. The slip ring drives the X-ray tube and the detector 102 to rotate under a drive of the driving mechanism 3, while the X-ray tube 101 may generate an X-ray beam under a control of the controller. The X-ray beam penetrates the inspected object 300 moving on the transmission device 200 and is irradiated onto a detector array 102. The detector array 102 converts the X-ray beam received into an electrical signal and transmits the electrical signal to a data acquisition module. An image recognition module receives data of the data acquisition module, reconstructs the data received and generates image data. The image data generated is transmitted to the computer for recognition and inspection of the inspected object.

In an exemplary embodiment, referring to FIG. 1 to FIG. 8, the roller 511 of the at least one roller assembly 51 has an annular recess 515 surrounding an outer periphery of the roller, and the annular rail 4 is at least partially engaged with the recess 515 to prevent a movement of the slip ring in an axial direction. In an alternative exemplary embodiment, the annular rail has a rail recess arranged in a circumferential direction, and the roller of the at least one roller assembly is at least partially engaged into the rail recess.

In the CT scanning apparatus according to the embodiments of the present disclosure, the driving mechanism 3 is used to drive the slip ring to rotate with respect to the supporting frame 1. For example, two annular rails 4 are provided on the slip ring, the roller 511 of each group of supporting devices 5 is engaged with the annular rail 4 to rotatably support the slip ring, and the annular rail 4 is at least partially engaged with the recess 515 of the roller 511. In this way, the movement of the slip ring in the axial direction may be prevented. An outer diameter of the annular roller 511 is significantly smaller than an outer diameter of the slip ring 2, for example, the outer diameter of the annular roller 511 is one-fifteenth to one-eighth of the outer diameter of the slip ring 2, and a plurality of annular rollers 511 may prevent the movement of the slip ring in the axial direction while supporting the slip ring.

In an exemplary embodiment, as shown in FIG. 6, a buffer layer 41 is provided on a surface of the annular rail 4 in contact with the roller 511. In another embodiment, a buffer layer is provided on a surface of the roller 511 in contact with the annular rail 4, or buffer layers are provided on both surfaces of the annular rail 4 and the roller 511. A thermal expansion of the annular rail 4 may be absorbed by providing the buffer layer, and a damage caused by a contact between the roller 511 and the annular rail may be prevented.

In an exemplary embodiment, as shown in FIG. 6, the recess 515 of each roller 511 has a substantially V-shaped cross section, and a cross section of the annular rail 4 has a shape cooperating with the recess 515. For example, an included angle between two sides of the recess 515 of the substantially V-shape is 85 to 95 degrees, preferably 90 degrees. The roller 511 may perform an axial positioning on the annular rail by providing the cross sections of the recess 515 and the annular rail to be of substantial V-shapes. When the recess and the annular rail are worn out, a beam surface of a beam emitted by the X-ray tube of the CT scanning apparatus will not be axially displaced.

In an exemplary embodiment, as shown in FIG. 3 to FIG. 5, each roller assembly 51 further includes: a base 513 mounted on the support frame 1 through a bolt assembly 512; and a support shaft 514 fixedly mounted on the base 513, and the roller 511 is rotatably mounted on the support shaft 514. In this way, the support frame 1 achieves a support for the slip ring through the base 513 and the roller 511. In an exemplary embodiment, as shown in FIG. 3 and FIG. 4, the roller of the support device located on the left side in FIG. 3 is provided with a recess, and the recess 515 of the roller 511 cooperates with the annular rail 4. The roller includes at least one of a V-shaped bearing wheel, an elastic V-shaped bearing wheel, a cylindrical bearing wheel, and an elastic cylindrical bearing wheel. The support frame 1 includes at least two upright frames and at least two horizontal frames, and the upright frames and the horizontal frames are combined by welding.

In an exemplary embodiment, as shown in FIG. 3 and FIG. 5, an outer circumferential surface of the roller of the support device located on the right side in FIG. 3 is provided to be substantially flat. That is, the roller 511 is in smooth contact with the annular rail 4. It should be understood that in this case, the support device located on the right side is mainly used to support the slip ring 2. In an alternative embodiment, the roller of the support device located on the right side may be designed with a recess engaged with the annular rail.

In an exemplary embodiment, as shown in FIG. 3 to FIG. 7, each group of support devices 5 includes four roller assemblies 51 arranged at equal intervals on the outer periphery of the slip ring 2. However, the embodiments of the present disclosure are not limited to this. According to actual needs, each group of support devices may be provided with 3 or 5 roller assemblies arranged in a star shape, and distances between two adjacent isolation assemblies may be unequal.

In an exemplary embodiment, the base 513 of each roller assembly 51 is mounted with a roller 511. In an alternative embodiment, in the bases of the four roller assemblies 51, the two bases located on an upper portion of an axis of the slip ring 2 are respectively mounted with one roller, and the two bases located on a lower portion of the axis of the slip ring are respectively mounted with two rollers. In this way, the bases located on the lower portion of the axis of the slip ring are all mounted with two rollers, which may support a weight of the slip ring and prevent the movement of the slip ring in the axial direction.

In an exemplary embodiment, as shown in FIG. 3 and FIG. 5, in the bases of the four roller assemblies, the two bases 513 located on the upper portion of the axis of the slip ring 2 are respectively mounted with an elastic mechanism, and the elastic mechanisms 516 are configured so that rollers mounted on the two bases 513 elastically abut on the annular rail 4. The thermal expansion of the annular rail 4 may be absorbed by providing the elastic mechanism 516, thereby reducing an adverse effect caused by a processing and mounting error on the entire CT scanning apparatus.

In an exemplary embodiment, as shown in FIG. 3 to FIG. 7, annular mounting seats 6 are respectively provided at two ends of the slip ring 2. For example, the mounting seat 6 has a substantial flange shape and is mounted at an end portion of the slip ring 2 through a plurality of bolt components. The driving mechanism 3 is configured to drive one of the mounting seats 6, e.g., the mounting seat 6 on the right side in FIG. 3 to rotate.

In an exemplary embodiment, as shown in FIG. 3 to FIG. 7, the driving mechanism 3 includes: a stator assembly mounted on the support frame 1; and a rotor assembly mounted on the mounting seat 6, the stator assembly being electromagnetically coupled with the rotor assembly, so as to drive the rotor assembly to rotate with respect to the stator assembly. Further, the stator assembly includes a stator 31 having a cylinder shape which is mounted on the support frame 1, and an induction coil 32 wound on the stator 31. On the other hand, the rotor assembly includes a rotor 33 having a cylinder shape and a permanent magnet 34, one end of the rotor 33 is mounted on the mounting seat 6 and is located on an inner side of the stator 31. The permanent magnet 34 is mounted on an outer side of the rotor 33 and is electromagnetically coupled with the induction coil 32. In this way, the stator assembly and the rotor assembly form a motor, and the stator 31 directly drives the slip ring to rotate. Therefore, such driving manner of the slip ring is called "self-driven".

In an alternative embodiment, the driving mechanism may include a motor and a conveyor belt, such as a multi-ribbed belt, driven by the motor and surrounding the slip ring. In another embodiment, the driving mechanism includes a motor and a driving wheel driven by the motor and being in contact with the outer periphery of the slip ring.

In an exemplary embodiment, as shown in FIG. 3 and FIG. 4, one of the two annular rails 4 is mounted on the mounting seat 6 through a plurality of bolt components.

In an exemplary embodiment, as shown in FIG. 6 and FIG. 7, at least two lubricating mechanisms 7 are provided on the support frame 1, and the lubricating mechanisms 7 are configured to apply a lubricant to the annular rail 4. Further, each lubricating mechanism 7 includes: a container 71 mounted on the support frame 1 and configured to contain the lubricant; an applicator 72, such as a brush, configured to apply the lubricant to the annular rail 4; and a transmission pipe 72 connected between the container 71 and the applicator 72 so as to transmit the lubricant in the container 71 to the applicator 72. The lubricating mechanism 7 further incudes a regulator 74 configured to regulate a flow of the lubricant. In this way, the regulator 74 may be operated as required to adjust an amount of the lubricant. A small amount lubricant may be used to remove a heat from the annular rail, and beneficial for reducing a friction and lowering a noise.

In an exemplary embodiment, the lubricating mechanism 7 further includes a support stand 75. The support stand 75 is mounted on the support frame 1, and the applicator 72 is mounted on the support stand 75, so as to stably support the applicator 72.

In the CT scanning apparatus according to the embodiments of the present disclosure, a cantilever structure used to support the slip ring may be omitted by using a plurality of rollers having a small external diameter. The rollers may be easily replaced when being damaged, and the cost is low. As there is no requirement for a pressing force between friction pairs formed by the roller and the annular rail, the weight of the CT scanning apparatus is reduced, and an overall structure thereof is also more compact. The roller having the substantially V-shaped recess cooperates with the annular rail having the substantially V-shaped protrusion to axially position the slip ring. When the roller and the annular rail are worn out, the beam surface of the beam will not be axially displaced.

Further, in the CT scanning apparatus according to the embodiments of the present disclosure, a self-driven manner is used for the slip ring, which may simplify the drive system, improve the drive efficiency, reduce a startup time of the rotation of the slip ring, reduce a drive failure rate, and improve a convenience of apparatus maintenance.

Those skilled in the art may understand that the embodiments described above are all exemplary, and those skilled in the art may make improvements thereto. Structures described in the various embodiments may be freely combined without a structural or principal conflict.

Although the present disclosure is described with reference to the accompanying drawings, the embodiments disclosed in the accompanying drawings are intended to illustrate the preferred embodiments of the present disclosure, and should not be construed as limiting the present disclosure. Although some embodiments of the inventive concept of the present disclosure have been shown and described, those of ordinary skill in the art will understand that modifications may be made to these embodiments without departing from the principle and spirit of the general inventive concept of the present disclosure. The scope of the present disclosure is defined by the claims and their equivalents.

## Claims

1. A CT scanning apparatus (100), comprising:
a support frame (1);
a slip ring (2);
at least two annular rails (4) mounted on an outer periphery of the slip ring;
a driving mechanism (3) configured to drive the slip ring to rotate with respect to the support frame; and
at least two groups of support devices (5), wherein each group of support devices comprises at least two roller assemblies (51) surrounding the annular rail and configured to support the slip ring,
wherein each roller assembly comprises at least one roller (511), and the at least one roller is mounted on the support frame and engaged with the annular rail so as to support the slip ring, and
wherein a roller of at least one roller assembly of the at least two roller assemblies is engaged with the annular rail to prevent a movement of the slip ring in an axial direction.

2. The CT scanning apparatus according to claim 1, wherein the roller of the at least one roller assembly has an annular recess (515) surrounding an outer periphery of the roller, and the annular rail is at least partially engaged into the recess.

3. The CT scanning apparatus according to claim 1, wherein the annular rail has a rail recess arranged in a circumferential direction, and the roller of the at least one roller assembly is at least partially engaged into the rail recess.

4. The CT scanning apparatus of any one of claims 1-3, wherein the recess of each roller has a substantially V-shaped cross section, and a cross section of the annular rail has a shape cooperating with the recess.

5. The CT scanning apparatus according to claim 4, wherein an included angle between two sides of the concave having the substantially V-shape is 85 to 95 degrees, preferably 90 degrees.

6. The CT scanning apparatus according to any one of claims 1-5, wherein each roller assembly further comprises:
a base (513) mounted on the support frame; and
a support shaft (514) fixedly mounted on the base, wherein the roller is rotatably mounted on the support shaft.

7. The CT scanning apparatus according to claim 6, wherein each group of support devices comprises four roller assemblies arranged at equal intervals on the outer periphery of the slip ring.

8. The CT scanning apparatus according to claim 7, wherein the base of each roller assembly is mounted with a roller.

9. The CT scanning apparatus according to claim 7, wherein in the four bases of the four roller assemblies, two bases located on an upper portion of an axis of the slip ring are respectively mounted with one roller, and two bases located on a lower portion of the axis of the slip ring are respectively mounted with two rollers.

10. The CT scanning apparatus according to claim 7, wherein in the four bases of the four roller assemblies, two bases located on an upper portion of an axis of the slip ring are respectively mounted with an elastic mechanism, and the elastic mechanisms are configured so that the rollers mounted on the two bases elastically abut on the annular rail.

11. The CT scanning apparatus according to any one of claims 1-10, wherein an annular mounting seat (6) is provided at one end of the slip ring, and the driving mechanism is configured to drive the mounting seat to rotate.

12. The CT scanning apparatus according to claim 11, wherein the driving mechanism comprises:
a stator assembly mounted on the support frame; and
a rotor assembly mounted on the mounting seat, wherein the stator assembly is electromagnetically coupled with the rotor assembly so as to drive the rotor assembly to rotate with respect to the stator assembly.

13. The CT scanning apparatus according to claim 12, wherein the stator assembly comprises:
a stator (31) mounted on the support frame, wherein the stator has a cylinder shape; and
an induction coil (32) wound on the stator, and
wherein the rotor assembly comprises:
a rotor (33) having a cylinder shape, wherein one end of the rotor is mounted on the mounting seat and located on an inner side of the stator; and
a permanent magnet (34) mounted on an outer side of the rotor and electromagnetically coupled with the induction coil.

14. The CT scanning apparatus according to claim 13, wherein one of the two annular rails is mounted on the mounting seat.

15. The CT scanning apparatus according to any one of claims 1-14, wherein at least two lubricating mechanisms (7) are provided on the support frame, and the lubricating mechanism is configured to apply a lubricant to the annular rail.

16. The CT scanning apparatus according to claim 15, wherein each lubricating mechanism comprises:
a container (71) mounted on the support frame and configured to contain the lubricant;
an applicator (72) configured to apply the lubricant to the annular rail; and
a transmission pipe (72) connected between the container and the applicator so as to transmit the lubricant in the container to the applicator.

17. The CT scanning apparatus according to claim 16, wherein the lubricating mechanism further comprises a regulator (74) configured to regulate a flow of the lubricant.

18. The CT scanning apparatus according to claim 16 or 17, wherein the lubricating mechanism further comprises a support stand (75), the support stand is mounted on the support frame, and the applicator is mounted on the support stand.

19. The CT scanning apparatus according to any one of claims 1-19, wherein a buffer layer (41) is provided on a surface of at least one of the annular rail and the roller.
